# EUROPEAN PATENT SPECIFICATION

(11) **EP 3 544 945 B1**
(45) Date of publication and mention of the grant of the patent: **03.11.2021**
(21) Application number: 17801741.4
(22) Date of filing: 27.11.2017
(51) Int. Cl.: C07C 37/50, C07C 39/07

(54) **PROCESS FOR PREPARING (POLY)ALKYLATED PHENOLS**
VERFAHREN ZUR HERSTELLUNG VON (POLY)ALKYLIERTEN PHENOLEN
PROCÉDÉ DE PRÉPARATION DES PHÉNOLS (POLY)ALKYLÉS

(30) Priority: 28.11.2016 EP 16200943
(43) Date of publication of application: 02.10.2019
(73) Proprietor: DSM IP Assets B.V., 6411 TE Heerlen (NL)
(72) Inventor: LETINOIS, Ulla, 4303 Kaiseraugst (CH); MEDLOCK, Jonathan Alan, 4303 Kaiseraugst (CH); LEHMANN, Hajo, 4303 Kaiseraugst (CH)
(74) Representative: Dux, Roland
(86) International application number: PCT/EP2017/080545
(87) International publication number: WO 2018/096152

(56) References cited:
- DE-C- 432 151
- US-A- 2 841 624
- VAN DUZEE EZ AL.: "Hydrogenation and Hydrogenolysis of Ethers", J. AM. CHEM. SOC., vol. 57, 1935, pages 147-151, XP002770028,

## Description

### Technical Field

The present invention relates to the field of production of (poly)alkylphenols and their uses.

### Background of the invention

(Poly)alkylphenols are an important class of chemicals which are of great industrial importance. Particularly methylphenols (=cresols), dimethylphenols and trimethylphenols are industrially important products or starting materials.

Cresols and dimethylphenols can be extracted from coal tar, which is a non-renewable source and critical in view of labour health.

A key intermediate for the synthesis of α-tocopherol is 2,3,6-trimethylphenol. 2,3,6-trimethylphenol is traditionally synthesized from m-cresol or 3,6-xylenol by catalytic alkylation with methanol or by condensation of diethyl ketone with methyl vinyl ketone or crotonaldehyde. These procedures are very complex and non-selective. Therefore, there is a big need for an alternative industrial process which allows the production of (poly)alkylphenols in a highly efficiency and a safe way.

DE 432 151 C discloses in example 6 the hydrogenation of N-2-oxybenzyl aniline ortho-anilinomethyl-substituted phenols in the presence of palladium to aniline and cresols.

### Summary of the invention

Therefore, the problem to be solved by the present invention is to provide an alternative efficient way of manufacturing (poly)alkylphenols.

Surprisingly, it has been found that a process according to claim 1 solves this problem. The process offers a very high flexibility in obtaining different desired (poly)alkylphenols in high yield and low environmental impact.

### Detailed description of the invention

In a first aspect, the invention relates to a process of reducing a compound of formula (I') 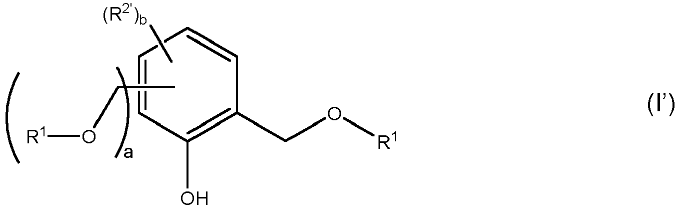 by a reducing agent in the presence of a heterogeneous metal catalyst to yield a compound of formula (A') 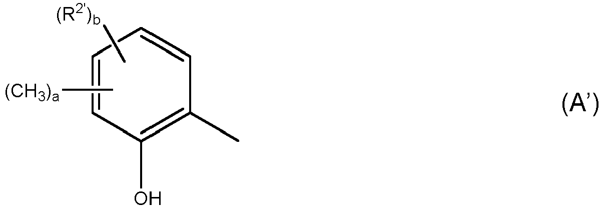
wherein the metal is selected from the group consisting of Ni, Fe, Ir, Pd, Pt, Rh and Ru;
and R¹ represents a C₁₋₁₁-alkyl group, which optionally comprises at least one carbon-carbon double bond and/or at least one nitrogen or oxygen atom, or a C₄₋₇-cycloalkyl group;
and wherein R^{2'} represents a C₁₋₁₀-alkyl group
and a and b are integer of 0, 1, 2, 3 or 4 with the proviso that the sum of a and b is a value of 0 - 4, wherein a=0.

In case where b is an integer > 1, the groups R^{2'} may be identical or different from each other. It is preferred that in such a case the groups R^{2'} are identical.

In the present document, the groups bound to the aromatic ring have been chosen to be represented in the following way in the formulae

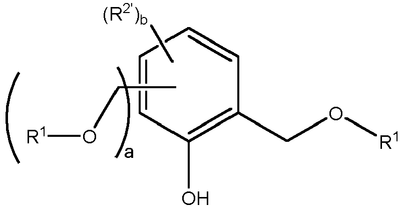

There are b times groups of R^{2'} and a times groups of CH₂OR¹ bound directly to the aromatic ring.

The position of said groups can be at any carbon of the aromatic ring except the ones which are already occupied by OH and CH₂OR¹.

It is important to note that the position of the CH₂OR¹ in ortho-position to the OH group as required in said formulae is essential for the present invention. For example, it has been found that compounds having no CH₂OR¹ in ortho-position to the OH group, such as 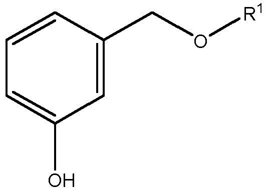 do not undergo a reaction in the desired manner.

In case of b being an integer of 1 or larger, it is preferred that the position of the additional group(s) of CH₂OR¹ is (are) in the meta position to the OH group.

A "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group.

In case identical labels for symbols or groups are present in several formulae, in the present document, the definition of said group or symbol made in the context of one specific formula applies also to other formulae which comprises said same label.

The expression "process of preparation" is a synonym for "method of preparation" and can be used interchangeable to each other.

### Compound of formula (I')

In the present process compound of formula (I') is reduced to yield compound of formula (A').

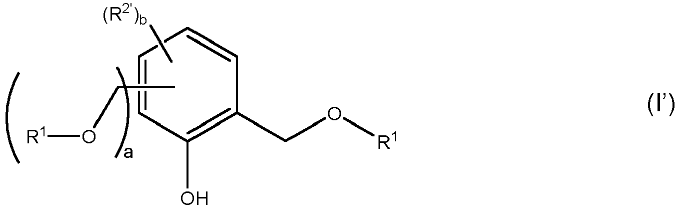

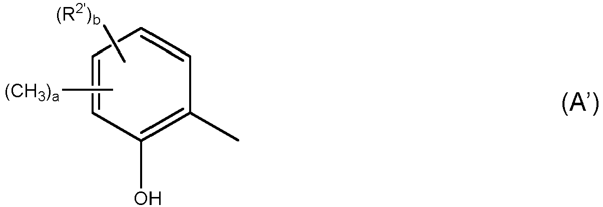

Compound of formula (I') can be prepared by any suitable method, for example, from salicylaldehyde or its derivatives by the methods disclosed by Cavitt et al (J. Org. Chem, 1962, 27, 1211) or Panten et al (WO 2007/065755A1)

R¹ represents a C₁₋₁₁-alkyl group, which optionally comprises at least one carbon-carbon double bond and/or at least one nitrogen or oxygen atom, or a C₄₋₇-cycloalkyl group. It is preferred that R¹ represents a C₁₋₆-alkyl group, preferably a C₁₋₃-alkyl group, more preferably a methyl or ethyl or isopropyl group, even more preferably a methyl group.

In a further embodiment, R¹ represents a C₁₋₁₁-alkyl group, which comprises at least one nitrogen or oxygen atom. Particular examples for such groups of R¹ are 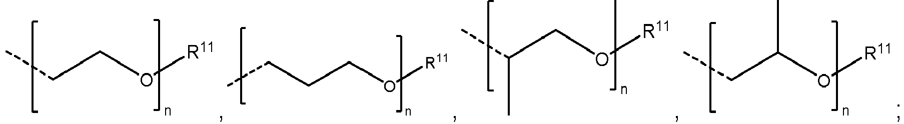 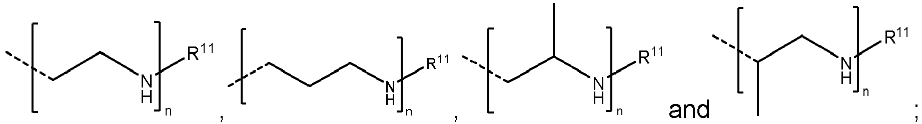 and, n being an integer of 1 - 5, particularly of 1-3; and R¹¹ being a C₁₋₆-alkyl alkyl group, particularly a methyl group.

R^{2'} represents a C₁₋₁₀-alkyl group. It is preferred that R^{2'} represents a C₁₋₆-alkyl group, preferably a C₁₋₃-alkyl group, more preferably a methyl or ethyl or isopropyl group, even more preferably a methyl group.

It is preferred that the compound of formula (I') is a compound of formula (I) and the compound of formula (A') is of a compound of formula (A) (= 2,3,6-trimethylphenol)

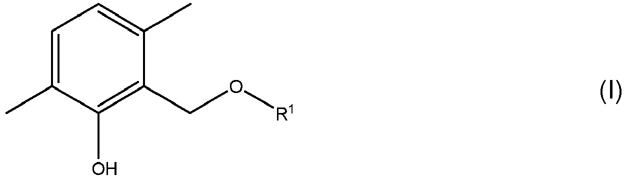

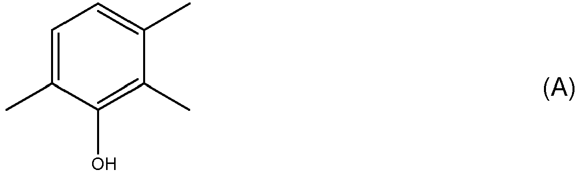

It is also preferred that compound of formula (I') is either a compound of formula (I") or (I'") and the compound of formula (A') is correspondingly a compound of formula (A") or (A'")

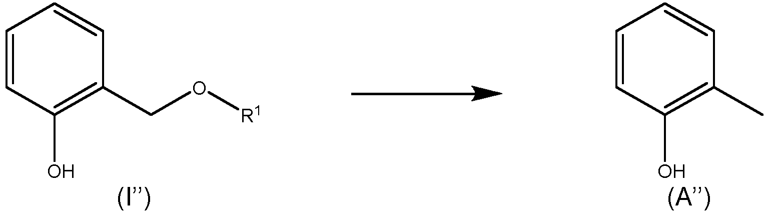

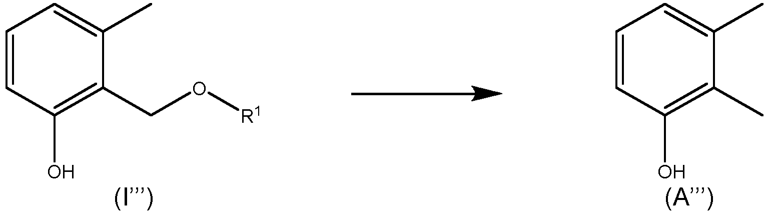

### Reducing agent

Compound of formula (I') is reduced in the above process by a reducing agent.

In one preferred embodiment of the invention the reducing agent is molecular hydrogen. In other words, in one embodiment the compound of formula (I') is hydrogenated by molecular hydrogen in the presence of a heterogeneous metal catalyst.

In another preferred embodiment of the invention the reducing agent is a transfer hydrogenation agent. In other words, in another embodiment the compound of formula (I'), is transfer hydrogenated by a transfer hydrogenation agent. Said transfer hydrogenation agent is preferably formic acid and/or a formic acid salt.

### Heterogeneous metal catalyst

The compound of formula (I') is reduced by a reducing agent in the presence of a heterogeneous metal catalyst wherein the metal is selected from the group consisting of Ni, Fe, Ir, Pd, Pt, Rh and Ru.

It is preferred that the metal of the heterogeneous metal catalyst comprises at least one of the metals selected from the group consisting of Ni, Fe, Ir, Pd, Pt and Rh. The heterogeneous metal catalyst can be a catalyst comprising more than one of the mentioned metals.

Particularly, the heterogeneous metal catalyst comprises Pd and Pt as metal.

It is preferred that the metal of the heterogeneous metal catalyst is palladium.

A wide variety of heterogeneous metal catalysts are known. Particularly useful are heterogeneous metal catalyst which are on a carrier or support material. Such carrier material is particularly a solid material having a high surface area, to which the metal is affixed. The support may be inert or participate in the catalytic reactions. Typical supports/carrier material include various kinds of carbon, alumina, and silica. The preferred support/carrier material is carbon.

The heterogeneous metal catalyst may also be affixed or immobilized on a surface of a larger object typically in form of a structured packing element which might be a part of the reactor in which the reduction takes place or an element which is inserted into said reactor. This structured packing element may be a dumped packing, a knit, an open-celled foam structure, preferably made of plastic, for example polyurethane or melamine resin, or ceramic, or a structured packing element, as already known in principle, i.e. by its geometric shape, from distillation and extraction technology. However, for the purposes of the present invention, structured packings in principle have a substantially smaller hydraulic diameter, frequently by a factor of from 2 to 10, than comparable internals in the field of distillation and extraction technology. Useful structured packing elements are in particular metal fabric packings and wire fabric packings, for example of the design Montz A3, Sulzer BX, DX and EX. Instead of metal fabric packings, it is also possible to use structured packings made of other woven, knitted or felted materials. Further useful structured packings are of flat or corrugated sheets, preferably without perforation, or other relatively large orifices, for example corresponding to the designs Montz BI or Sulzer Mellapak. The structured packings made of expanded metal are also advantageous, for example structured packings of the type Montz BSH.

It is preferred that the heterogeneous metal catalyst is a palladium catalyst, particularly a palladium on carbon catalyst (Pd/C).

The catalytic metal loading (i.e. weight metal / weight (metal+carrier)) is typically between 1 to 20%, preferably between 4 and 11%, more preferably between 4 and 6% by weight. A very preferred heterogeneous metal catalyst is palladium on carbon catalyst (Pd/C) of which 5 % by weight is palladium (i.e. loading = 5%).

The reduction can be carried out in the presence or absence of solvents. Suitable solvents are particularly those in which the compound of formula (I') is soluble. Particularly, the solvent is an organic solvent, preferably a solvent selected from the group consisting of alcohols, preferably the alcohol of formula R¹OH, or ethers, preferably dialkyl ether or cyclic ethers, preferably tetrahydrofuran. R¹OH is the alcohol which is formed in the reduction reaction as mentioned above.

Furthermore, suitable additives may be added to the reaction mixture of the compound of formula (I'), the heterogeneous metal catalyst and the reducing agent. Particularly, organic acids, preferably acetic acid, can be added to the mixture before or during the reduction.

The reduction is performed typically at temperatures of between 20 and 80°, particularly between 30 and 50°C, and preferably under a pressure. In case of use of molecular hydrogen, it is preferred that the reduction is carried out under a pressure of hydrogen of between 2 and 20 bar, preferably of between 3 and 7 bar.

The reduction is performed in a suitable vessel. The reduction can be made batchwise or continuously. Suitable reactors for industrial scale are known.

It is preferred that the weight ratio of the heterogeneous metal catalyst to the compound of formula (I') is 0.01% to 20%, particularly 1 % to 10%.

It has been observed that the process as described above yields efficiently a compound of formula (A')

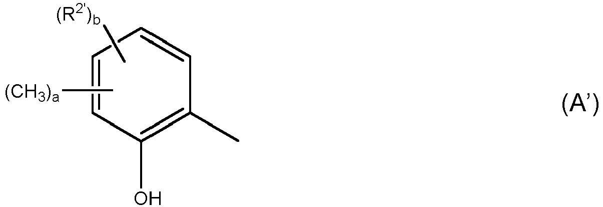

Such substituted phenols can be used in a variety of fields, either directly or indirectly. On the one hand they may be used directly, such as antioxidants, on the other hand they are important starting materials for synthesizing compounds useful in pharmaceuticals, food or feed supplements, cosmetics, or flavors and flagrances.

A particularly relevant example of this is, for some specific compounds of formula (A'), the manufacture of the compound of formula (C') from the compound of formula (A').

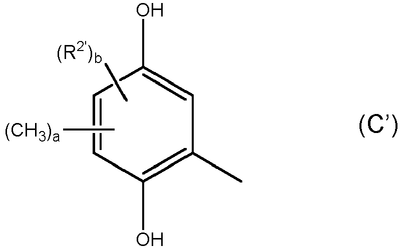

Therefore, in a further aspect, the invention relates to a process for the manufacture of the compound of formula (C') from compound of formula (I') comprising the following steps
a') reducing the compound of formula (I') by the process as described above in detail to yield a compound of formula (A');
   with the proviso
   that a and b are integer of 0, 1, 2 or 3
   and
   that the sum of a and b is a value of 0 - 3;
   and
   that the carbon in the para position to the OH group carries a hydrogen atom; 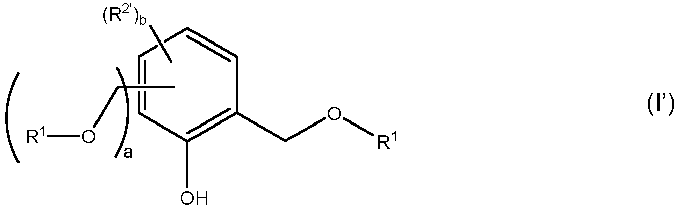 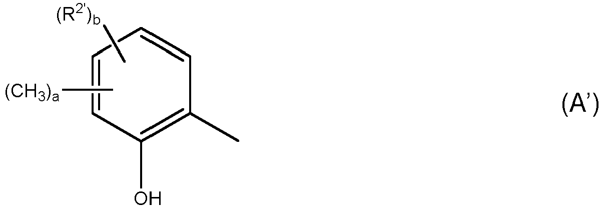
b') oxidizing the compound of formula (A') to yield a compound of formula (B') by an oxidizing agent 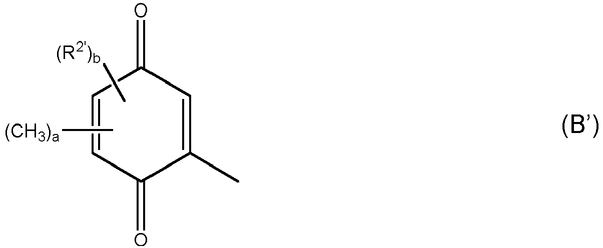
c') reducing the compound of formula (B') to yield a compound of formula (C') by a reducing agent

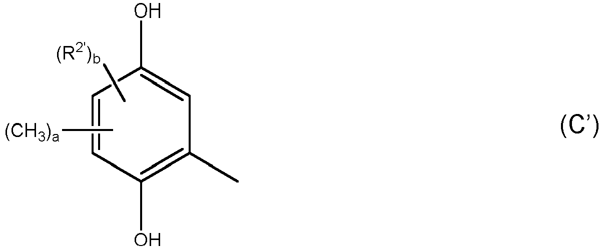

wherein R¹ represents a C₁₋₁₁-alkyl group, which optionally comprises at least one carbon-carbon double bond and/or at least one nitrogen or oxygen atom, or a C₄₋₇-cycloalkyl group;
and wherein R^{2'} represents a C₁₋₁₀-alkyl group
and a=0.

In step b') the compound of formula (A') is oxidized to the compound of formula (B'). The oxidation is preferably carried out by any suitable method known in the art, for example: with air using salcomine as catalyst in ethanol according to the procedure published by A. Stocker, W.-D. Woggon, A. Rüttimann, Helv. Chim. Acta 1993, 76, 1729-1738, or by copper and molecular oxygen as described in EP 0127888 A1 or by the use of a heteropoly acid and oxygen as described by Kholdeeva et al in Journal of Molecular Catalysis, 75 (1992) 235-244.

In step c) the compound of formula (B') is reduced to compound of formula (C'). The reduction can be performed by hydrogenation or be achieved with sodium dithionite in water according to the method as disclosed by K. Sato, Y. Fujima, A. Yamada Bull. Chem. Soc. Jap. 1968, 41, 442-444.

Compounds of formula (C') can be used directly in various fields or can be used as important starting materials for the synthesis of compounds suitable for the synthesis of compounds useful in pharmaceuticals, food or feed supplements, cosmetics, or flavors and flagrances.

Particularly important is the use of compound of formula (C') as antioxidant, in plastics, adhesives, inks, composites, or in organisms.

A particular important compound of formula (A') is the compound of formula (A)

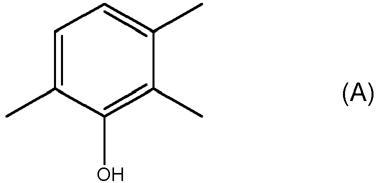

This compound can be obtained by the process as disclosed above starting from a compound of formula (I'-a)

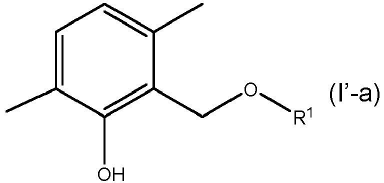

The compound of formula (A) is particularly important as it can be used as a starting material for the synthesis of α-tocopherol.

Therefore, in a further aspect, the invention relates to a process for the manufacture of compound of formula (D) from compound of formula (I) comprising the steps
a) hydrogenate compound of formula (I) by the process, as disclosed above in detail, to yield compound of formula (A); 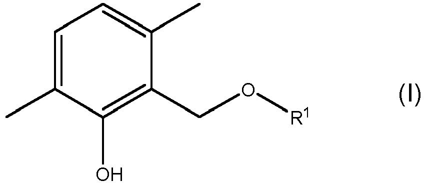 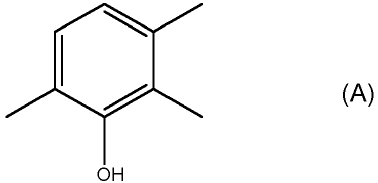 wherein R¹ represents a C₁₋₁₁-alkyl group, which optionally comprises at least one carbon-carbon double bond and/or at least one nitrogen or oxygen atom, or a C₄₋₇-cycloalkyl group;
b) oxidizing the compound of formula (A) to yield a compound of formula (B) by an oxidizing agent 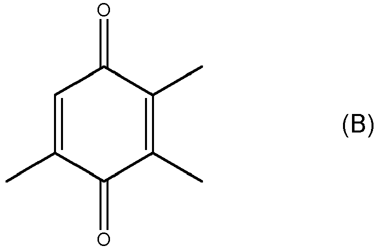
c) reducing the compound of formula (B) to yield a compound of formula (C) by a reducing agent 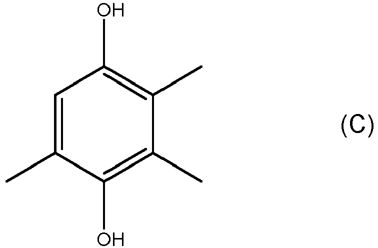
d) condensing isophytol with the compound of formula (C) to yield a compound of formula (D);

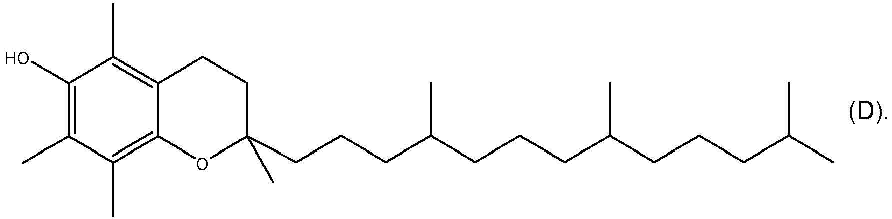

The conditions and production procedures of step a'), b') or c'), respectively, correspond to those of step a), b) or c), respectively, as disclosed above.

In step d) isophytol (=3,7,11,15-tetramethylhexadec-1-en-3-ol) is condensed with a compound of formula (C) to yield a compound of formula (D). Isophytol and a compound of formula (C), described as step d), is known by the person skilled in the art. For this condensation a series of catalysts may be used such as ZnCl₂/mineral acid, BF₃/AlCl₃, Fe/HCI, trifluoroacetic acid or boric acid/carboxylic acid as well as indium(III) or scandium(III) salts as disclosed in WO 2005/121115 A1. Furthermore, suitable catalysts are heteropoly acids, particularly 12-tungstophosphoric acid or 12-tungstosilicic acid such as disclosed in EP 0 970 953 A1.

A further aspect of the invention relates to a use of a heterogeneous metal catalyst in the reduction of the compound of formula (I'), as mentioned above in detail, by a reducing agent 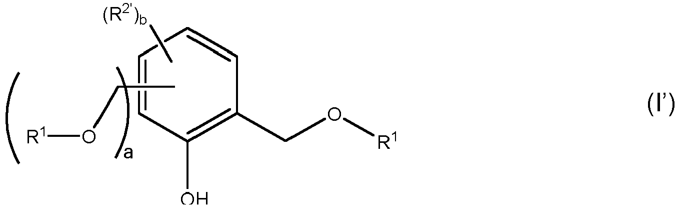 to yield a compound of formula (A') 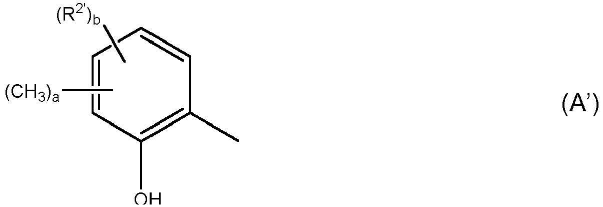
wherein R¹ represents a C₁₋₁₁-alkyl group, which optionally comprises at least one carbon-carbon double bond and/or at least one nitrogen or oxygen atom, or a C₄₋₇-cycloalkyl group;
and wherein R^{2'} represents a C₁₋₁₀-alkyl group
and a and b are integer of 0, 1, 2, 3 or 4 with the proviso that the sum of a and b is a value of 0 - 4;
characterized in that the metal is selected from the group consisting of Ni, Fe, Ir, Pd, Pt, Rh and Ru
wherein a=0.

### Examples

The present invention is further illustrated by the following non-limiting experiments.

### Formation of 2-methyl phenol (=o-cresol)

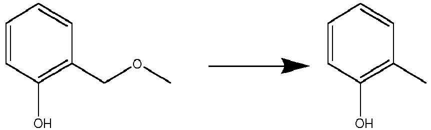

2-(Methoxymethyl)phenol (60 % purity. Sigma-Aldrich) ("MMP") was dissolved in a solvent as indicated in table 1 and 5% by weight palladium on carbon (Evonik E 101 N/D, 20 mg) was added. The reactor was sealed and purged three times with nitrogen and three times with hydrogen. The mixture was stirred at 40 °C under 5 bar hydrogen pressure for a time as indicated in table 1. The pressure was released, the catalyst was removed by filtration and the mixture evaporated under reduced pressure to give a crude product which was analysed by GC-MS.

| | **MMP** | **Solvent (amount)** | **Additive** | **Time (hours)** | **Conversion (%)** | **Yield (GC area%)** |
|---|---|---|---|---|---|---|
| ***1*** | 400 mg | MeOH (4.0 g) | None | 4 | >99 | 83 |
| ***2*** | 200 mg | MeOH (2.0 g) | Acetic acid (0.2 g) | 19 | >99 | 69 |
| ***3*** | 200 mg | THF (2.0 g) | None | 0.5 | >99 | 67 |

### Formation of 2,3,6-trimethylphenol (example 4)

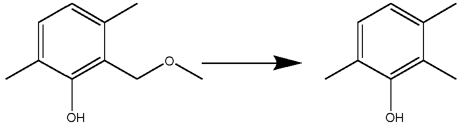

2-(methoxymethyl)-3,6-dimethylphenol (32 mg) was dissolved in methanol (1.0 g) and 5% palladium on carbon (Evonik E 101 N/D, 3 mg) was added. The reactor was sealed and purged three times with nitrogen and three times with hydrogen. The mixture was stirred at 40 °C under 5 bar hydrogen pressure for 22 hours. An additional 3 mg of 5% palladium on carbon was added and the mixture was stirred at 40 °C under 5 bar hydrogen pressure for a further 22 hours. The pressure was released, the catalyst was removed by filtration and the mixture evaporated under reduced pressure to give a crude product. GC-MS analysis showed full conversion and 98.5% purity product (yield 98%).

### Comparitive examples

5-methyl-1,3-dihydroisobenzofuran-4-ol or 3-(methoxymethyl)phenol, respectively, (both having no methoxymethyl group in the ortho-position to the OH-group) have been submitted to the same reduction conditions as for example ***4*** with the object to yield 2,3,6-trimethylphenol, or m-cresol, respectively. However, in both cases, the desired product has not been detected.

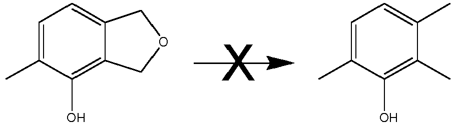

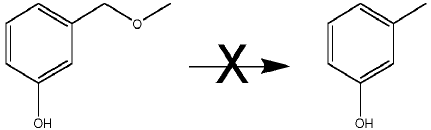

## Claims

1. A process of reducing a compound of formula (I') 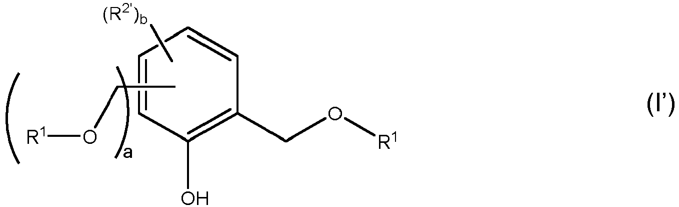 by a reducing agent in the presence of a heterogeneous metal catalyst to yield a compound of formula (A') 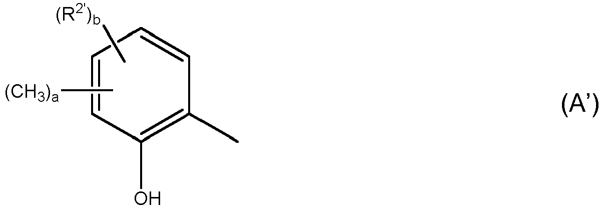
wherein the metal is selected from the group consisting of Ni, Fe, Ir, Pd, Pt, Rh and Ru;
and R¹ represents a C₁₋₁₁-alkyl group, which optionally comprises at least one carbon-carbon double bond and/or at least one nitrogen or oxygen atom, or a C₄₋₇-cycloalkyl group;
and wherein R^{2'} represents a C₁₋₁₀-alkyl group
and a and b are integer of 0, 1, 2, 3 or 4 with the proviso that the sum of a and b is a value of 0 - 4; wherein a = 0.

2. The process according to claim 1, **characterized in that** the compound of formula (I') is a compound of formula (I) and the compound of formula (A') is a compound of formula (A)

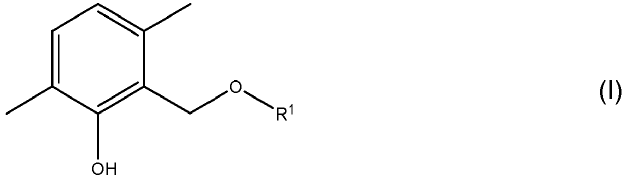

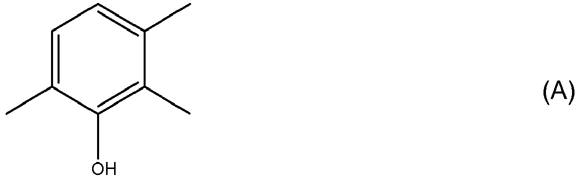

3. The process according to claim 1, **characterized in that** the compound of formula (I') is either a compound of formula (I") or (I'") and the compound of formula (A') is correspondingly a compound of formula (A") or (A"')

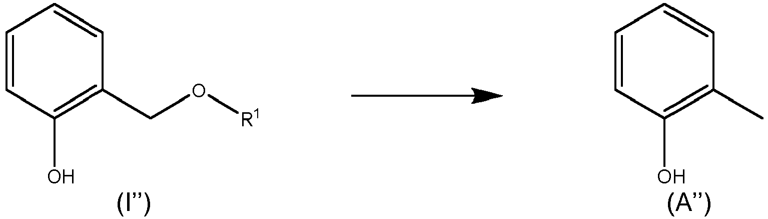

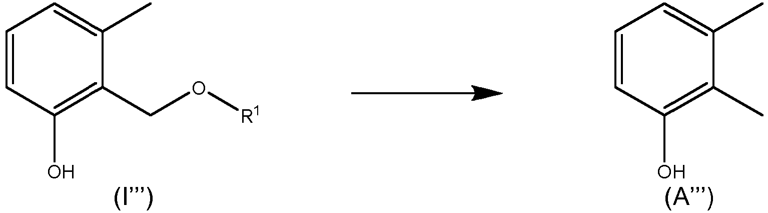

4. The process according to any one of the preceding claims 1 - 3, **characterized in that** the compound of formula (I') is hydrogenated by molecular hydrogen in the presence of a heterogeneous metal catalyst.

5. The process according to any one of the preceding claims 1 - 3, **characterized in that** the compound of formula (I') is transfer hydrogenated by a transfer hydrogenation agent, preferably by formic acid and/or a formic acid salt, in the presence of a heterogeneous metal catalyst.

6. The process according to any one of the preceding claims, **characterized in that** the heterogeneous metal catalyst comprises at least one of the metals selected from the group consisting of Ni, Fe, Ir, Pd, Pt and Rh.

7. The process according to any one of the preceding claims, **characterized in that** the heterogeneous metal catalyst is a palladium catalyst, particularly a palladium on carbon catalyst.

8. The process according to any one of the preceding claims, **characterized in that** R¹ represents a C₁₋₆-alkyl group, preferably a C₁₋₃-alkyl group, more preferably a methyl group.

9. The process according to any one of the preceding claims, **characterized in that** R^{2'} represents a C₁₋₆-alkyl group, preferably a C₁₋₃-alkyl group, more preferably a methyl group.

10. The process according to any one of the preceding claims, **characterized in that** the weight ratio of heterogeneous metal catalyst to compound of formula (I') is 0.01% to 20%, particularly 1 % to 10 %.

11. A process for the manufacture of the compound of formula (C') from compound of formula (I') comprising the following steps
a') reducing the compound of formula (I') by the process according to any one of the preceding claims 1-10 to yield a compound of formula (A');
with the proviso
that a and b are integer of 0, 1, 2 or 3
and
that the sum of a and b is a value of 0 - 3;
and
that the carbon in the para position to the OH group carries a hydrogen atom; 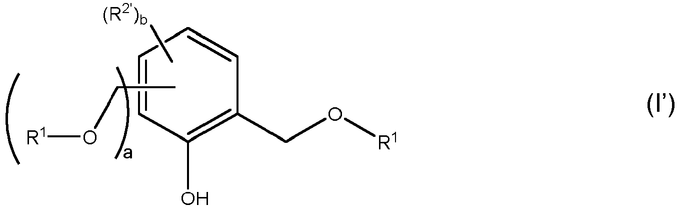 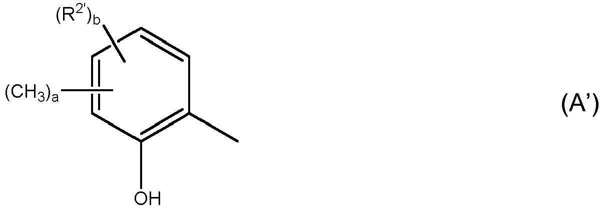
b') oxidizing the compound of formula (A') to yield a compound of formula (B') by an oxidizing agent 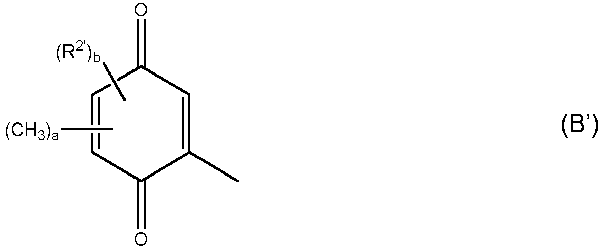
c') reducing the compound of formula (B') to yield a compound of formula (C') by a reducing agent 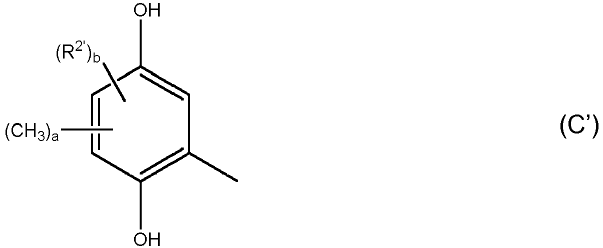
wherein R¹ represents a C₁₋₁₁-alkyl group, which optionally comprises at least one carbon-carbon double bond and/or at least one nitrogen or oxygen atom, or a C₄₋₇-cycloalkyl group;
and wherein R^{2'} represents a C₁₋₁₀-alkyl group
and a=0

12. A process for the manufacture of compound of formula (D) from compound of formula (I) comprising the steps
a) hydrogenate compound of formula (I) by the process according to claim 2 or any one of the preceding claims 4-10 to yield compound of formula (A); 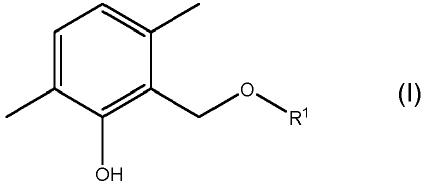 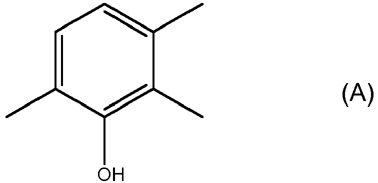 wherein R¹ represents a C₁₋₁₁-alkyl group, which optionally comprises at least one carbon-carbon double bond and/or at least one nitrogen or oxygen atom, or a C₄₋₇-cycloalkyl group;
b) oxidizing the compound of formula (A) to yield a compound of formula (B) by an oxidizing agent 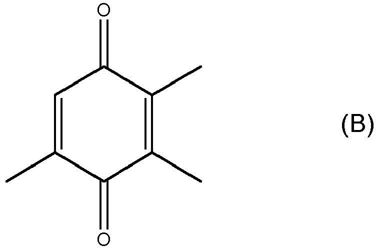
c) reducing the compound of formula (B) to yield a compound of formula (C) by a reducing agent 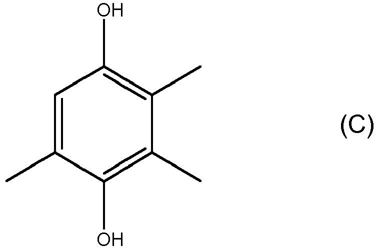
d) condensing isophytol with the compound of formula (C) to yield a compound of formula (D); 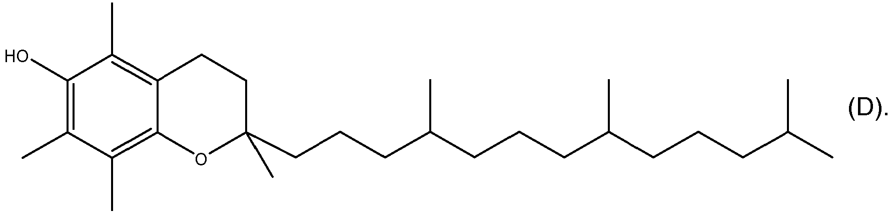
wherein a=0.

13. A use of a heterogeneous metal catalyst in the reduction of a compound of formula (I') by a reducing agent 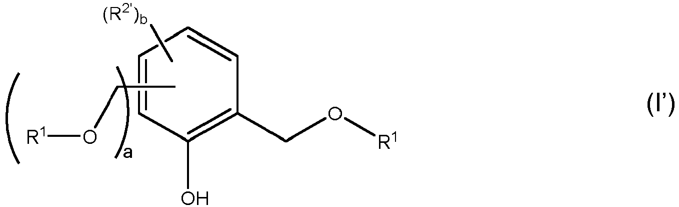 to yield a compound of formula (A') 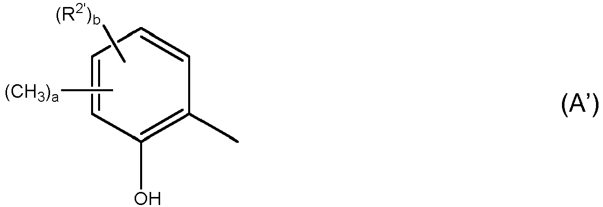
wherein R¹ represents a C₁₋₁₁-alkyl group, which optionally comprises at least one carbon-carbon double bond and/or at least one nitrogen or oxygen atom, or a C₄₋₇-cycloalkyl group;
and wherein R^{2'} represents a C₁₋₁₁-alkyl group
and a and b are integer of 0, 1, 2, 3 or 4 with the proviso that the sum of a and b is a value of 0 - 4;
**characterized in that** the metal is selected from the group consisting of Ni, Fe, Ir, Pd, Pt, Rh and Ru
wherein a=0.

## Patentansprüche

1. Verfahren zur Reduktion einer Verbindung der Formel (I') 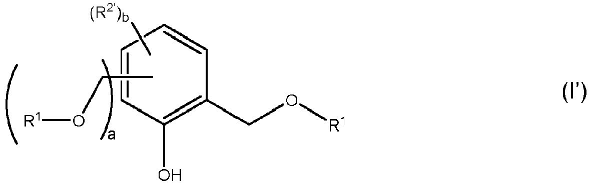 mit einem Reduktionsmittel in Gegenwart eines heterogenen Metallkatalysators zu einer Verbindung der Formel (A') 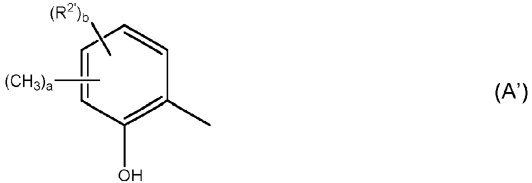
wobei das Metall aus der Gruppe bestehend aus Ni, Fe, Ir, Pd, Pt, Rh und Ru ausgewählt ist und R¹ für eine C₁₋₁₁-Alkylgruppe, die gegebenenfalls mindestens eine Kohlenstoff-Kohlenstoff-Doppelbindung und/oder mindestens ein Stickstoff- oder Sauerstoffatom umfasst, oder eine C₄₋₇-Cycloalkylgruppe steht
und wobei R^{2'} für eine C₁₋₁₀-Alkylgruppe steht und a und b für eine ganze Zahl mit einem Wert von 0, 1, 2, 3 oder 4 stehen, mit der Maßgabe, dass die Summe von a und b ein Wert von 0-4 ist; wobei a = 0.

2. Verfahren nach Anspruch 1, **dadurch gekennzeichnet, dass** es sich bei der Verbindung der Formel (I') um eine Verbindung der Formel (I) handelt und es sich bei der Verbindung der Formel (A') um eine Verbindung der Formel (A) handelt:

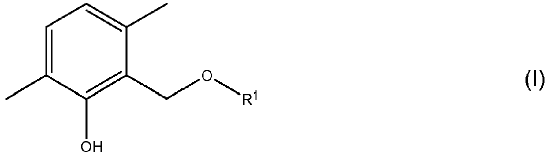

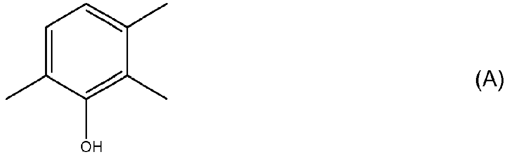

3. Verfahren nach Anspruch 1, **dadurch gekennzeichnet, dass** es sich bei der Verbindung der Formel (I') um eine Verbindung der Formel (I'') oder (I''') handelt und es sich bei der Verbindung der Formel (A') entsprechend um eine Verbindung der Formel (A'') oder (A''') handelt:

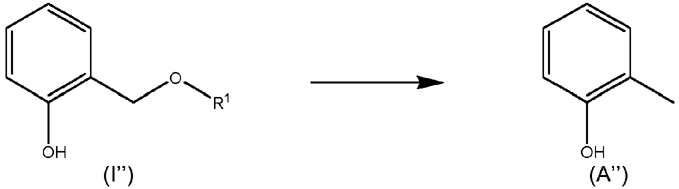

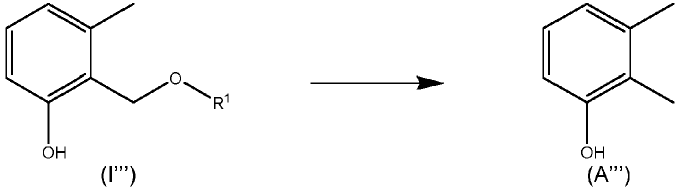

4. Verfahren nach einem der vorhergehenden Ansprüche 1-3, **dadurch gekennzeichnet, dass** die Verbindung der Formel (I') mit molekularem Wasserstoff in Gegenwart eines heterogenen Metallkatalysators hydriert wird.

5. Verfahren nach einem der Ansprüche 1-3, **dadurch gekennzeichnet, dass** die Verbindung der Formel (I') mit einem Transferhydrierungsmittel, vorzugsweise mit Ameisensäure und/oder einem Ameisensäuresalz, in Gegenwart eines heterogenen Metallkatalysators transferhydriert wird.

6. Verfahren nach einem der vorhergehenden Ansprüche, **dadurch gekennzeichnet, dass** der heterogene Metallkatalysator mindestens eines der Metalle aus der Gruppe bestehend aus Ni, Fe, Ir, Pd, Pt und Rh umfasst.

7. Verfahren nach einem der vorhergehenden Ansprüche, **dadurch gekennzeichnet, dass** es sich bei dem heterogenen Metallkatalysator um einen Palladiumkatalysator, insbesondere einen Palladium-auf-Kohle-Katalysator handelt.

8. Verfahren nach einem der vorhergehenden Ansprüche, **dadurch gekennzeichnet, dass** R¹ für eine C₁₋₆-Alkylgruppe, vorzugsweise eine C₁₋₃-Alkylgruppe, weiter bevorzugt eine Methylgruppe, steht.

9. Verfahren nach einem der vorhergehenden Ansprüche, **dadurch gekennzeichnet, dass** R^{2'} für eine C₁₋₆-Alkylgruppe, vorzugsweise eine C₁₋₃-Alkylgruppe, weiter bevorzugt eine Methylgruppe, steht.

10. Verfahren nach einem der vorhergehenden Ansprüche, **dadurch gekennzeichnet, dass** das Gewichtsverhältnis von heterogenem Metallkatalysator zu Verbindung der Formel (I') 0,01 % bis 20 %, insbesondere 1 % bis 10 %, beträgt.

11. Verfahren zur Herstellung der Verbindung der Formel (C') aus der Verbindung der Formel (I'), das die folgenden Schritte umfasst:
a') Reduzieren der Verbindung der Formel (I') gemäß dem Verfahren nach einem der vorhergehenden Ansprüche 1-10 zu einer Verbindung der Formel (A');
mit der Maßgabe,
dass a und b für eine ganze Zahl mit einem Wert von 0, 1, 2 oder 3 stehen
und
dass die Summe von a und b ein Wert von 0-3 ist
und
dass das Kohlenstoffatom in der para-Position zur OH-Gruppe ein Wasserstoffatom trägt; 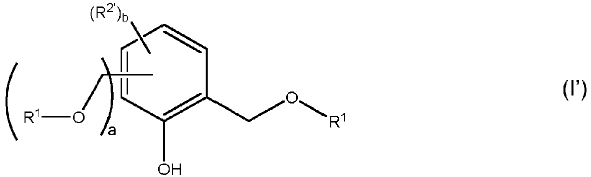 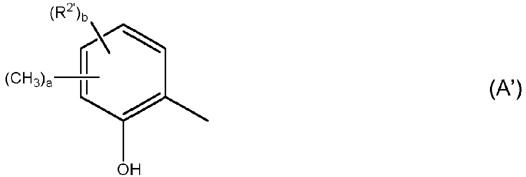
b') Oxidieren der Verbindung der Formel (A') zu einer Verbindung der Formel (B') mit einem Oxidationsmittel: 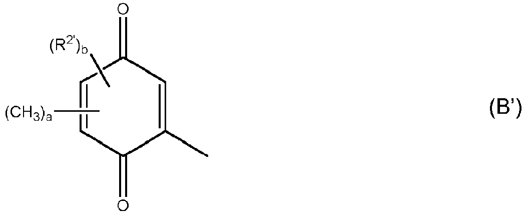
c') Reduzieren der Verbindung der Formel (B') zu einer Verbindung der Formel (C') mit einem Reduktionsmittel: 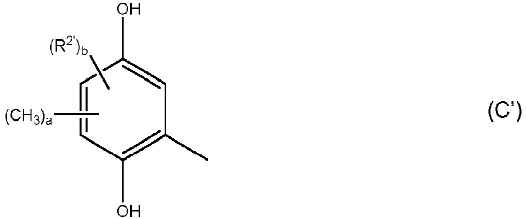
wobei R¹ für eine C₁₋₁₁-Alkylgruppe, die gegebenenfalls mindestens eine Kohlenstoff-Kohlenstoff-Doppelbindung und/oder mindestens ein Stickstoff- oder Sauerstoffatom umfasst, oder eine C₄₋₇-Cycloalkylgruppe steht
und wobei R^{2'} für eine C₁₋₁₀-Alkylgruppe steht und a = 0.

12. Verfahren zur Herstellung der Verbindung der Formel (D) aus der Verbindung der Formel (I), das folgende Schritte umfasst:
a) Hydrieren der Verbindung der Formel (I) gemäß dem Verfahren nach Anspruch 2 oder einem der vorhergehenden Ansprüche 4-10 zu einer Verbindung der Formel (A); 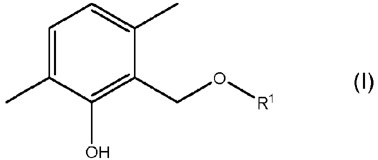 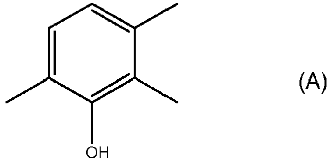 wobei R¹ für eine C₁₋₁₁-Alkylgruppe, die gegebenenfalls mindestens eine Kohlenstoff-Kohlenstoff-Doppelbindung und/oder mindestens ein Stickstoff- oder Sauerstoffatom umfasst, oder eine C₄₋₇-Cycloalkylgruppe steht;
b) Oxidieren der Verbindung der Formel (A) zu einer Verbindung der Formel (B) mit einem Oxidationsmittel: 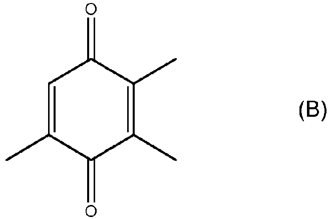
c) Reduzieren der Verbindung der Formel (B) zu einer Verbindung der Formel (C) mit einem Reduktionsmittel: 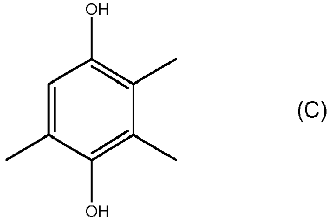
d) Kondensieren von Isophytol mit der Verbindung der Formel (C) zu einer Verbindung der Formel (D) ; 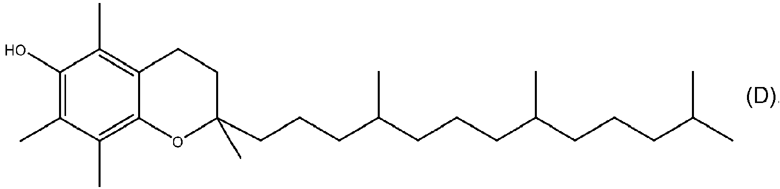
wobei a = 0.

13. Verwendung eines heterogenen Metallkatalysators bei der Reduktion einer Verbindung der Formel (I') mit einem Reduktionsmittel 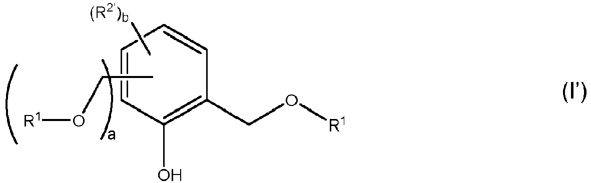 zu einer Verbindung der Formel (A') 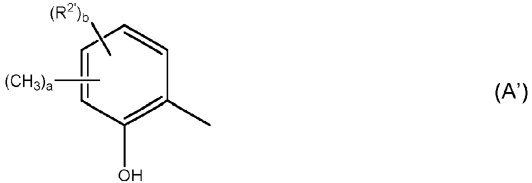
wobei R¹ für eine C₁₋₁₁-Alkylgruppe, die gegebenenfalls mindestens eine Kohlenstoff-Kohlenstoff-Doppelbindung und/oder mindestens ein Stickstoff- oder Sauerstoffatom umfasst, oder eine C₄₋₇-Cycloalkylgruppe steht
und wobei R^{2'} für eine C₁₋₁₁-Alkylgruppe steht und a und b für eine ganze Zahl mit einem Wert von 0, 1, 2, 3 oder 4 stehen, mit der Maßgabe, dass die Summe von a und b ein Wert von 0-4 ist;
**dadurch gekennzeichnet, dass** das Metall aus der Gruppe bestehend aus Ni, Fe, Ir, Pd, Pt, Rh und Ru ausgewählt ist,
wobei a = 0.

## Revendications

1. Procédé de réduction d'un composé de formule (I') 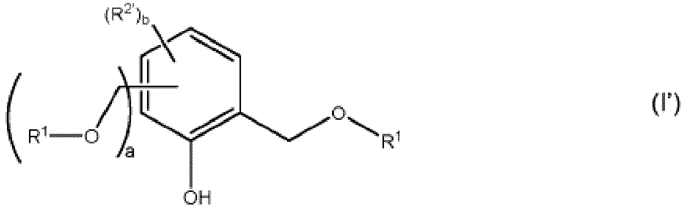 par un agent de réduction en la présence d'un catalyseur métallique hétérogène pour donner un composé de formule (A') 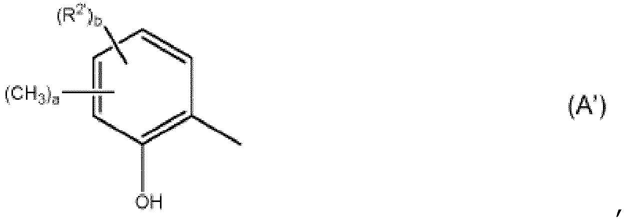
le métal étant choisi dans le groupe constitué par Ni, Fe, Ir, Pd, Pt, Rh et Ru ;
et R¹ représentant un groupe C₁₋₁₁-alkyle, qui comprend éventuellement au moins une double liaison carbone-carbone et au moins un atome d'azote ou d'oxygène, ou un groupe C₄₋₇-cycloalkyle ;
et R^{2'} représentant un groupe C₁₋₁₀-alkyle et a et b étant un entier parmi 0, 1, 2, 3 et 4, à la condition que la somme de a et b soit une valeur de 0 à 4 ; dans lequel a = 0.

2. Procédé selon la revendication 1, **caractérisé en ce que** le composé de formule (I') est un composé de formule (I) et le composé de formule (A') est un composé de formule (A)

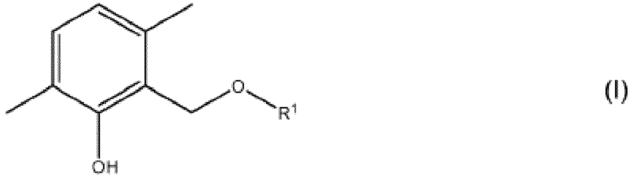

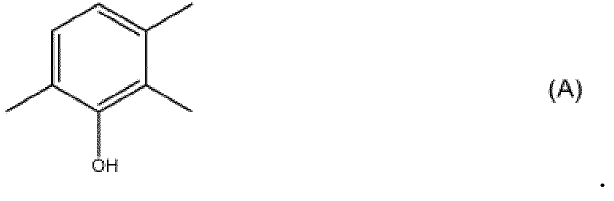

3. Procédé selon la revendication 1, **caractérisé en ce que** le composé de formule (I') est soit un composé de formule (I"), soit (I''') et le composé de formule (A') est de façon correspondante un composé de formule (A'') ou (A"')

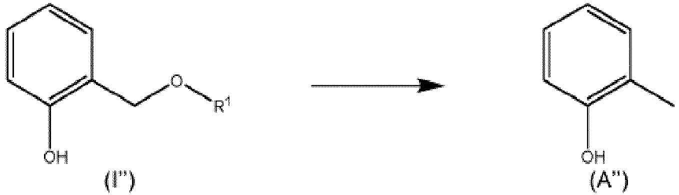

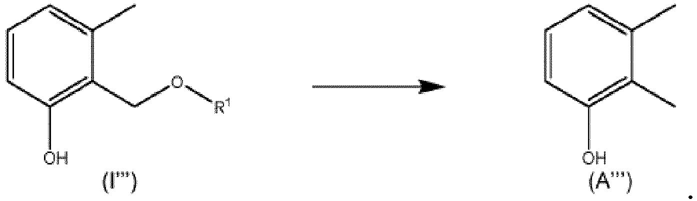

4. Procédé selon l'une quelconque des revendications précédentes 1 à 3, **caractérisé en ce que** le composé de formule (I') est hydrogéné par de l'hydrogène moléculaire en la présence d'un catalyseur métallique hétérogène.

5. Procédé selon l'une quelconque des revendications précédentes 1 à 3, **caractérisé en ce que** le composé de formule (I') est hydrogéné par transfert par un agent d'hydrogénation par transfert, préférablement par de l'acide formique et/ou un sel d'acide formique, en la présence d'un catalyseur métallique hétérogène.

6. Procédé selon l'une quelconque des revendications précédentes, **caractérisé en ce que** le catalyseur métallique hétérogène comprend au moins l'un des métaux choisis dans le groupe constitué par Ni, Fe, Ir, Pd, Pt et Rh.

7. Procédé selon l'une quelconque des revendications précédentes, **caractérisé en ce que** le catalyseur métallique hétérogène est un catalyseur au palladium, particulièrement un catalyseur de type palladium sur carbone.

8. Procédé selon l'une quelconque des revendications précédentes, **caractérisé en ce que** R¹ représente un groupe C₁₋₆-alkyle, préférablement un groupe C₁₋₃-alkyle, plus préférablement un groupe méthyle.

9. Procédé selon l'une quelconque des revendications précédentes, **caractérisé en ce que** R^{2'} représente un groupe C₁₋₆-alkyle, préférablement un groupe C₁₋₃-alkyle, plus préférablement un groupe méthyle.

10. Procédé selon l'une quelconque des revendications précédentes, **caractérisé en ce que** le rapport en poids du catalyseur métallique hétérogène sur le composé de formule (I') est de 0,01 % à 20 %, particulièrement 1 % à 10 %.

11. Procédé pour la fabrication du composé de formule (C') à partir du composé de formule (I') comprenant les étapes suivantes
a') réduction du composé de formule (I') par le procédé selon l'une quelconque des revendications précédentes 1 à 10 pour donner un composé de formule (A') ;
à la condition
que a et b soient des entiers parmi 0, 1, 2 et 3
et
que la somme de a et b soit une valeur de 0 à 3 ;
et
que le carbone dans la position para par rapport au groupe OH porte un atome d'hydrogène ; 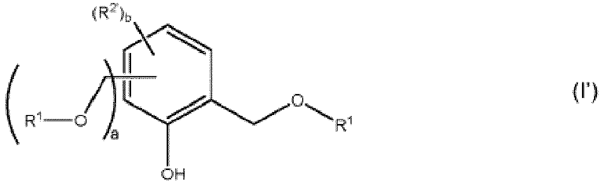 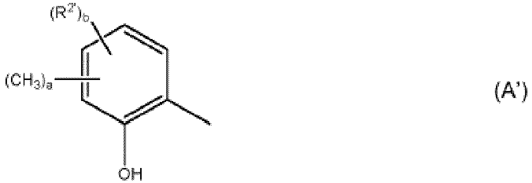
b') oxydation du composé de formule (A') pour donner un composé de formule (B') par un agent d'oxydation 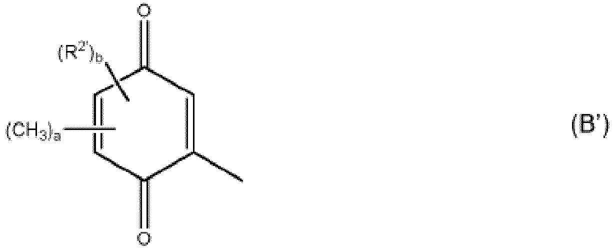
c') réduction du composé de formule (B') pour donner un composé de formule (C') par un agent de réduction 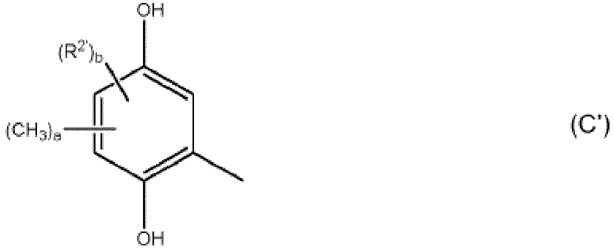
R¹ représentant un groupe C₁₋₁₁-alkyle, qui comprend éventuellement au moins une double liaison carbone-carbone et au moins un atome d'azote ou d'oxygène, ou un groupe C₄₋₇-cycloalkyle ;
et R^{2'} représentant un groupe C₁₋₁₀-alkyle et a = 0.

12. Procédé pour la fabrication d'un composé de formule (D) à partir du composé de formule (I) comprenant les étapes
a) hydrogénation du composé de formule (I) par le procédé selon la revendication 2 ou l'une quelconque des revendications précédentes 4 à 10 pour donner un composé de formule (A) ; 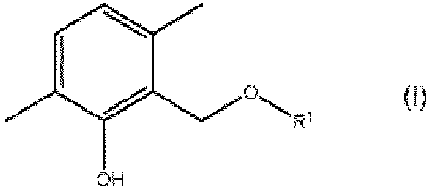 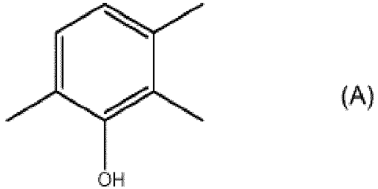 R¹ représentant un groupe C₁₋₁₁-alkyle, qui comprend éventuellement au moins une double liaison carbone-carbone et au moins un atome d'azote ou d'oxygène, ou un groupe C₄₋₇-cycloalkyle ;
b) oxydation du composé de formule (A) pour donner un composé de formule (B) par un agent d'oxydation 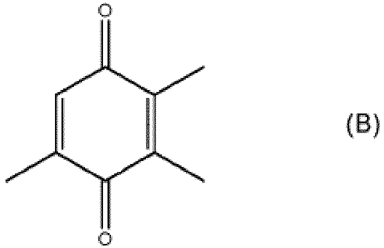
c) réduction du composé de formule (B) pour donner un composé de formule (C) par un agent de réduction 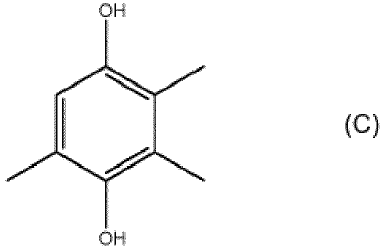
d) condensation d'isophytol avec le composé de formule (C) pour donner un composé de formule (D) ; 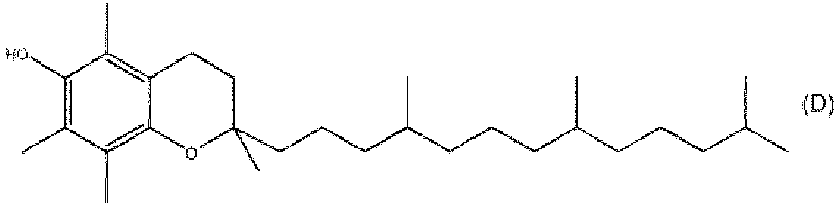
dans lequel a = 0.

13. Utilisation d'un catalyseur métallique hétérogène dans la réduction d'un composé de formule (I') par un agent de réduction 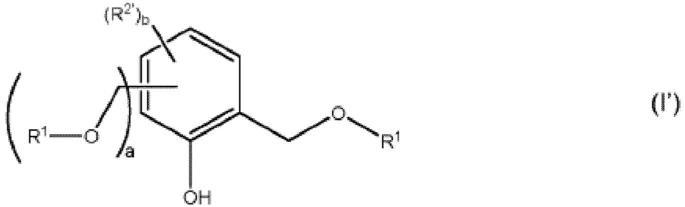 pour donner un composé de formule (A') 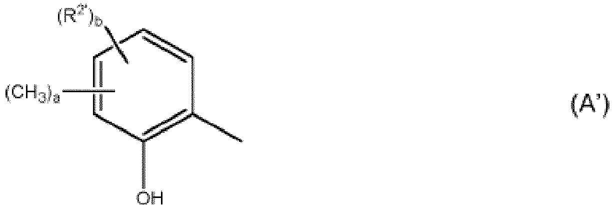
R¹ représentant un groupe C₁₋₁₁-alkyle, qui comprend éventuellement au moins une double liaison carbone-carbone et au moins un atome d'azote ou d'oxygène, ou un groupe C₄₋₇-cycloalkyle ;
et R^{2'} représentant un groupe C₁₋₁₀-alkyle et a et b étant un entier parmi 0, 1, 2, 3 et 4, à la condition que la somme de a et b soit une valeur de 0 à 4 ;
**caractérisée en ce que** le métal est choisi dans le groupe constitué par Ni, Fe, Ir, Pd, Pt, Rh et Ru, dans laquelle a = 0.
